(19) Europäisches Patentamt
European Patent Office
Office européen des brevets

(11) **EP 2 512 340 B1**

(12) **EUROPÄISCHE PATENTSCHRIFT**

(45) Veröffentlichungstag und Bekanntmachung des Hinweises auf die Patenterteilung:
**20.08.2014 Patentblatt 2014/34**

(51) Int Cl.:
*A61B 5/16* *(2006.01)*     *A61B 5/18* *(2006.01)*
*G08B 21/06* *(2006.01)*     *B60K 28/06* *(2006.01)*

(21) Anmeldenummer: **10787281.4**

(22) Anmeldetag: **23.11.2010**

(86) Internationale Anmeldenummer:
**PCT/EP2010/007090**

(87) Internationale Veröffentlichungsnummer:
**WO 2011/072794 (23.06.2011 Gazette 2011/25)**

(54) **VORRICHTUNG UND VERFAHREN ZUR BESTIMMUNG EINES VIGILANZZUSTANDES**

DEVICE AND METHOD FOR DETERMINING A VIGILANCE STATE

DISPOSITIF ET PROCÉDÉ DE DÉTERMINATION D'UN ÉTAT DE VIGILANCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.12.2009 DE 102009058459**

(43) Veröffentlichungstag der Anmeldung:
**24.10.2012 Patentblatt 2012/43**

(73) Patentinhaber: **Volkswagen AG**
**38436 Wolfsburg (DE)**

(72) Erfinder:
• **JUNGE, Mirko**
**38312 Cramme (DE)**
• **STAUBACH, Maria**
**01705 Freital (DE)**

(56) Entgegenhaltungen:
**EP-A1- 1 257 202     EP-A1- 1 929 950**

EP 2 512 340 B1

**Beschreibung**

Die Erfindung betrifft ein Verfahren und eine Vorrichtung zur Bestimmung eines Vigilanzzustandes eines Kraftfahrzeugführers.

Um die Sicherheit im Straßenverkehr durch Erkennen von Unaufmerksamkeiten oder Müdigkeit eines Kraftfahrzeugführers zu verbessern, werden Verfahren zur Bestimmung eines Vigilanzzustandes, also eines Aufmerksamkeitzustandes, des Kraftfahrzeugführers durchgeführt. Das Ziel dieser Verfahren ist es, Sicherheit im Straßenverkehr zu verbessern, indem ein ungewöhnliches Verhalten des Kraftfahrzeugführers detektiert und darauf aufmerksam gemacht wird. Das ungewöhnliche Verhalten kann hierbei beispielsweise durch Unaufmerksamkeit oder Müdigkeit entstehen. Zumeist wird durch Müdigkeit das Reaktionsvermögen des Kraftfahrzeugführers derart beeinflusst, dass Reflexe oder Reaktionen auf bestimmte Fahrsituationen langsamer, zeitverzögerter und/oder unsicherer erfolgen.

Die EP 1 257 202 B1 offenbart ein Verfahren zur Übermittlung eines Impulses an ein Fahrzeug während der Fahrt. Der Impuls wird hierbei mittels eines oder mehrerer Auslöser ausgelöst, wobei der Fahrer auf den Impuls üblicherweise spontan und unterbewusst reagiert. Der Impuls wird hierbei auf die Steuerung (oder auf ein anderes Teil, mit welchem der Fahrer üblicherweise in Kontakt ist) übermittelt. Weiter wird eine Antwort des Fahrers mittels eines oder mehrerer Sensoren, beispielsweise Lenkwinkel-, Drehmoment-, Beschleunigungskraft- oder Augenbewegungssensor detektiert. Weiter offenbart die Druckschrift, dass der Impuls und die Antwort verglichen werden, um Schlüsse bezüglich einer Geistesgegenwart des Fahrers zu ziehen, wobei die Geistesgegenwart dem Unterschied zwischen dem Impuls und der Antwort umgekehrt proportional ist. Die Druckschrift offenbart hierzu, dass die Geistesgegenwart des Kraftfahrzeugführers auf zwei verschiedene Arten detektiert werden kann. In einer ersten Alternative wird eine Differenz zwischen dem Impuls und der Antwort über eine bestimmte Zeitdauer aufintegriert. Je kleiner das Ergebnis der Integration, desto besser kann der Kraftfahrzeugführer auf Störungen reagieren. In einer zweiten Alternative wird eine Amplitude einer Fliehkraft in einer lateralen Richtung ausgewertet. Zusätzlich kann eine Reaktionszeit des Fahrers als Zeitdifferenz zwischen zwei verschiedenen Zeitpunkten bestimmt werden, wobei sich ein erster Zeitpunkt T1 dann ergibt, wenn der Impuls einen vorbestimmten Schwellwert und ein zweiter Zeitpunkt dann ergibt, wenn die Reaktion einen zweiten vorbestimmten Schwellwert überschreitet. Hierbei ergibt sich nachteilig, dass die schwellwertbasierte Dedektion des ersten und des zweiten Zeitpunktes zur Reaktionszeitbestimmung rauschanfällig ist. Ein Messrauschen, wie es zum Beispiel von den vorhergehend genannten Sensoren erzeugt wird, kann die Detektion einer Reaktionszeit des Kraftfahrzeugführers verfälschen.

Aus der EP 1 929 950 A1 ist ein Verfahren zur Bestimmung eines Vigilanzzustandes eines Kraftfahrzeugführers bekannt, wobei ein Rauschsignal erzeugt und ein Reaktionssignal des Kraftfahrzeugführers bestimmt wird und der Vigilanzzustand aus einem zeitlichen Verlauf des Rauschsignals und einem zeitlichen Verlauf des Reaktionssignales bestimmt wird.

Es stellt sich das technische Problem, ein Verfahren und eine Vorrichtung zur Bestimmung eines Vigilanzzustandes eines Kraftfahrzeugsführers zu schaffen, welche eine verbesserte, insbesondere zuverlässigere, Bestimmung des Vigilanzzustandes ermöglichen.

Die Lösung des technischen Problems ergibt sich aus den Merkmalen der unabhängigen Ansprüche 1 und 10. Weitere vorteilhafte Ausgestaltungen der Erfindung ergeben sich aus den Unteransprüchen.

Vorgeschlagen wird ein Verfahren zur Bestimmung eines Vigilanzzustandes eines Kraftfahrzeugführers. In dem Verfahren wird ein Rauschsignal erzeugt und ein von dem Rauschsignal abhängiges Zusatzmoment auf eine Lenkung, insbesondere auf eine Lenksäule eines Kraftfahrzeugs aufgebracht. Das Rauschsignal ist hierbei ein künstlich erzeugtes Signal. Vorzugsweise ist das Rauschsignal ein mittelwertfreies Signal. Weiter vorzugsweise ist das Rauschsignal ein Zufallssignal oder ein pseudo-zufälliges Signal. Weiter vorzugsweise weist das Rauschsignal kleine Amplituden auf, wobei kleine Amplituden ein Lenkverhalten und ein Fahrverhalten des Kraftfahrzeugs nicht oder nur unwesentlich beeinflussen. Das Rauschsignal kann hierbei beispielsweise von einem Lenkungssteuergerät der, beispielsweise elektromechanischen, Lenkung erzeugt werden. Selbstverständlich kann das Rauschsignal auch von weiteren, vorzugsweise im Kraftfahrzeug vorhandenen, Steuereinheiten erzeugt werden. Um das Zusatzmoment auf die Lenksäule aufzubringen, existieren mehrere Alternativen. In einer elektromechanischen Lenkung, bei der ein Servomotor ein Unterstützungsmoment auf eine Lenkzahnstange oder die Lenksäule aufbringt, kann das erfindungsgemäße Zusatzmoment mittels des Servomotors erzeugt werden. Hierfür kann das Rauschsignal beispielsweise auf einen Sollwert des Servomoments addiert werden. Hierbei versteht man unter dem Sollwert des Servomoments den Wert, den zum Beispiel der Servomotor auf die Lenkzahnstange aufbringen soll. Das Zusatzmoment kann alternativ auch mittels einer Einheit zur Erzeugung eines Zusatzmoments erzeugt und direkt oder indirekt, zum Beispiel mittels eines Getriebes, auf die Lenksäule aufgebracht werden. Vorstellbar ist zum Beispiel, dass eine Lenkung einen weiteren Servomotor umfasst, wobei der weitere Servomotor ausschließlich zur Erzeugung des Zusatzmomentes dient. Ebenso ist vorstellbar, gegebenenfalls hydraulische Aktoren einer Servolenkung zur Erzeugung des Zusatzmomentes zu nutzen.

Das Zusatzmoment kann hierbei direkt oder indirekt auf die Lenksäule aufgebracht werden. Ein indirektes Aufbringen bedeutet hierbei, dass das Zusatzmoment auch auf ein mechanisch mit der Lenksäule verbundenes Element der Lenkung aufgebracht werden kann. Beispielsweise kann das Zusatzmoment auf die Lenkzahnstange, mit welcher lenkbare Räder des Kraftfahrzeugs verbunden sind, aufgebracht werden. Die Lenkzahnstange ist hierbei mit der Lenksäule mechanisch gekoppelt, primär um Lenkbewegungen des Kraftfahrzeugführers auf die Lenkzahnstange zu übertragen. Aufgrund der mechanischen Kopplung wird das Zusatzmoment von der Lenkzahnstange auf die Lenksäule übertragen und kann, beispielsweise über ein Lenkrad, von dem Kraftfahrzeugführer haptisch erfasst werden. Allerdings kann das Zusatzmoment auch derart auf die Lenkung aufgebracht werden, dass es zwar zu einer Fahrzeugbewegungsänderung kommt, jedoch keine haptische Rückkopplung am Lenkrad über die Lenksäule spürbar ist.

Weiter wird ein zeitlicher Verlauf des Rauschsignals gespeichert. Ein zeitlicher Verlauf des Rauschsignals kann beispielsweise in einer Speichereinheit und/oder in dem Lenkungssteuergerät gespeichert werden.

Der Kraftfahrzeugführer wird das von dem Rauschsignal abhängige und auf die Lenksäule aufgebrachte Zusatzmoment haptisch erfassen und versuchen, dieses durch eine automatische, also nicht bewusste, Reaktion zu kompensieren. Gleiches gilt, wenn das Zusatzmoment nur eine Fahrzeugbewegungsänderung ohne haptische Rückwirkung erzeugt. Auch hier wird der Kraftfahrzeugführer automatisch versuchen, die Änderung zu kompensieren. Eine Reaktion des Kraftfahrzeugführers zur Kompensation des Zusatzmoments spiegelt sich hierbei in einem Reaktionssignal wieder. Erfindungsgemäß wird dieses Reaktionssignal des Kraftfahrzeugführers bestimmt. Das Reaktionssignal kann hierbei aus mindestens einer Zustandsgröße der Gruppe Handmoment, Gierrate, Lenkwinkel, Lenkwinkelgeschwindigkeit, Rotorlage des Servomotors und weiteren Zustandsgrößen bestimmt werden. Vorzugsweise wird das Reaktionssignal als das von dem Kraftfahrzeugführer mittels eines Lenkrades auf die Lenksäule aufgebrachte Handmoment bestimmt. Das Handmoment kann hierbei mittels eines Lenkmomentsensors erfasst werden. Auch ein zeitlicher Verlauf des Reaktionssignals wird, zum Beispiel in einer Speichereinheit und/oder in dem Lenkungssteuergerät, gespeichert.

Das derart bestimmte Reaktionssignal umfasst hierbei zwei Anteile. Ein erster Anteil wird aus einer Summe von Lenkbewegungen erzeugt, wobei die Summe von Lenkbewegungen aus einer Fahraufgabe des Kraftfahrzeugführers besteht. Die Fahraufgabe setzt sich zum Beispiel aus Anteilen einer Navigation, zum Beispiel Lenkmanöver um einer geplanten Route zu folgen, zusammen. Weiter umfasst die Fahraufgabe zum Beispiel Lenkmanöver, die aus verschiedenen Manövern des Kraftfahrzeugs, zum Beipiel Überholmanövern, resultieren. Weiter umfasst die Fahraufgabe Lenkmanöver zur Fahrzeugstabilisierung, zum Beispiel beim Überfahren einer Bodenwelle. Als zweiten Anteil umfasst das Reaktionssignal Lenkbewegungen des Kraftfahrzeugführers, die zur Kompensation des Zusatzmomentes dienen.

Erfindungsgemäß ist es nicht notwendig, den zweiten Anteil signaltechnisch von dem ersten Anteil zu trennen, um einen Vigilanzzustandes des Kraftfahrzeugführers zu bestimmen. Insbesondere ist es nicht notwendig, mittels zum Beispiel vorbestimmter Schwellwerte zu detektieren, ab welchem Zeitpunkt ein Reaktionssignal ausschließlich aus Anteilen zur Kompensation des Zusatzmomentes besteht.

Es ist jedoch vorstellbar, das Reaktionssignal zu filtern, um eine Signalqualität des Reaktionssignals zu verbessern. Mittels der Filterung muss jedoch keine Trennung des ersten und des zweiten Anteils des Reaktionssignals erreicht werden.

Der Vigilanzzustand des Kraftfahrzeugführers wird erfindungsgemäß aus einem zeitlichen Verlauf des Rauschsignals und einem zeitlichen Verlauf des Reaktionssignals bestimmt. Hierzu wird mindestens ein Parameter einer parametrisierten Übertragungsfunktion von Rauschsignal zu Reaktionssignal bestimmt. Die parametrisierte Übertragungsfunktion modelliert hierbei zumindest teilweise ein dynamisches Lenkverhalten des Kraftfahrzeugführers. Als Übertragungsfunktion sind lineare Übertragungsfunktionen oder nichtlineare Übertragungsfunktionen denkbar. Der mindestens eine Parameter der parametrisierten Übertragungsfunktion kann hierbei mittels eines Verfahrens zur Identifikation einer linearen oder nichtlinearen Übertragungsfunktion bestimmt werden.

Der Vigilanzzustand des Kraftfahrzeugführers wird dann abhängig von einem absoluten Wert des mindestens einen Parameters und/oder abhängig von einer relativen Änderung des mindestens einen Parameters zu mindestens einem früheren Parameter bestimmt. Der mindestens eine frühere Parameter ist hierbei zu einem früheren Zeitpunkt mittels des erfindungsgemäßen Verfahrens bestimmt worden. Wird der Vigilanzzustand abhängig von einem absoluten Wert des mindestens einen Parameters bestimmt, so ist vorstellbar, dass der mindestens eine Parameter einen vorbestimmten Schwellwert nicht über- oder unterschreiten darf. Hierbei liegt es im Ermessen des Fachmanns, einen geeigneten vorbestimmten Schwellwert, beispielsweise abhängig von einem Kraftfahrzeugmodell, zu bestimmen. Vorzugsweise erfolgt jedoch die Bestimmung des Vigilanzzustandes mittels einer Auswertung der relativen Änderung des mindestens einen Parameters. Ist eine relative Änderung des mindestens einen Parameters größer als ein vorbestimmter Schwellwert, zum Bespiel 10 Prozent, so kann eine Änderung des Vigilanzzustandes detektiert werden. Der mindestens eine frühere Parameter kann hierbei zu einem früheren Zeitpunkt bestimmt werden, der eine vorbestimmte Zeitdauer, beispielsweise 20 oder 30 Minuten, vor dem aktuellen Zeitpunkt liegt.

Abhängig von der absoluten und/oder relativen Änderung des mindestens einen Parameters kann ein kritischer oder unkritischer Vigilanzzustand des Kraftfahrzeugführers bestimmt werden. Wird ein kritischer Vigilanzzustand des Kraftfahrzeugführers bestimmt, so kann ein Warnsignal, zum Beispiel ein optisches oder akustisches Warnsignal, erzeugt oder eine Pausenempfehlung generiert werden.

Durch das erfindungsgemäße Verfahren ergibt sich in vorteilhafter Weise eine einfachere und zuverlässigere Bestimmung eines Vigilanzzustandes, wobei ein Vigilanzzustand insbesondere unabhängiger von einem Messrauschen bestimmt werden kann.

In einer weiteren Ausführungsform wird der mindestens eine Parameter der parametrisierten Übertragungsfunktion mittels einer Kreuzkorrelation des Rauschsignals mit dem Reaktionssignal bestimmt. Beispielsweise kann eine Übertragungsfunktion mittels

$$R_{YX}(t) = R_{XX}(t) * h(t) \hspace{4cm} \text{Formel 1}$$

bestimmt werden. Hierbei bezeichnet $R_{YX}(t)$ eine Kreuzkorrelationsfunktion des Rauschsignals mit dem Reaktionssignal und $R_{XX}(t)$ eine Autokorrelationsfunktion des Rauschsignals. Weiter bezeichnet h(t) die Impulsantwort einer linearen, zeitinvarianten Übertragungsfunktion, wobei sich $R_{YX}(t)$ mittels einer Faltung aus $R_{XX}(t)$ und der Impulsantwort ergibt. Aus der Impulsantwort h(t) kann in vorteilhafter Weise zuverlässig und in einer einfach zu implementierende Weise die Übertragungsfunktion bestimmt werden. Aus der Impulsantwort h(t) lässt sich mittels standardisierter Verfahren zur Systemindentifikation, beispielsweise einem Least-Squares-Verfahren, der mindestens eine Parameter der parametrisierten Übertragungsfunktion bestimmen.

In einer alternativen Ausführungsform wird der mindestens eine Parameter der parametrisierten Übertragungsfunktion mittels einer. Autokorrelation des Rauschsignals und einer Autokorrelation des Reaktionssignals bestimmt. Für eine lineare, zeitinvariante Übertragungsfunktion gilt

$$R_{YY}(t) = R_{XX}(t) * h(t) * h(-t) \hspace{3cm} \text{Formel 2.}$$

Hierbei bezeichnet Ryy (t) die Autokorrelationsfunktion des Reaktionssignals, $R_{XX}(t)$ die Autokorrelationsfunktion des Rauschsignals und h(t) wiederum die Inpulsantwort. Auch mittels dieser Ausführungsformkann in vorteilhafter Weise zuverlässig und in einer einfach zu implementierende Weise die Übertragungsfunktion bestimmt werden

Entsprechend bekannter Transformationsregeln können die in den Formeln 1 und 2 dargestellten Beziehungen auch von einem Zeitbereich in einen Frequenzbereich transformiert werden. Hierbei ergibt sich die gesuchte Übertragungsfunktion im Frequenzbereich aus einer entsprechenden Multiplikation der spektralen Leistungsdichtefunktionen $S_{XX}(\omega)$, $S_{XY}(\omega)$, $S_{YY}(\omega)$ mit der Übertragungsfunktion H(w) im Frequenzbereich.

Weiter ist vorstellbar, einen Korrelationskoeffizient der Kreuzkorrelationsfunktion $R_{XY}(t)$ auszuwerten. Hierbei kann der Kreuzkorrelationskoeffizient auch für eine Kreuzkorrelation des Reaktionssignals mit einem um eine vorbestimmte Zeitdifferenz vor dem Reaktionssignal liegenden Rauschsignal berechnet werden, wobei auch eine Berechnung für mehrere Zeitdifferenzen möglich ist. Werden mehrere Zeitdifferenzen ausgewertet , so entspricht beispielsweise die Zeitdifferenz zwischen Rausch- und Reaktionssignal, die dem maximalen Korrelationskoeffizienten entspricht, einer Verzögerungszeit des Kraftfahrzeugführers. Ein Vigilanzzustand kann in diesem Fall abhängig von der Zeitdifferenz und/oder dem absoluten Wert des Korrelationskoeffizienten und/oder einer Änderung des Korrelationskoeffizienten in Relation zu einem früher bestimmten Korrelationskoeffizienten bestimmt werden.

In einer weiteren Ausführungsform ist die parametrisierte Übertragungsfunktion eine lineare und/oder zeitinvariante Übertragungsfunktion. Vorzugsweise ist die parametrisierte Übertragungsfunktion eine lineare und zeitinvariante Übertragungsfunktion. Hierbei ist die lineare und/oder zeitinvariante Übertragungsfunktion in der Regel eine Näherung für das normalerweise nichtlineare dynamische Lenkverhalten des Kraftfahrzeugführers. Mittels der linearen und/oder zeitinvarianten Übertragungsfunktion wird das dynamische Lenkverhalten des Kraftfahrzeugführers um einen Arbeitspunkt des nichtlinearen Systems als annähernd linear modelliert. Hierzu werden die Betrachtungszeiträume, also die Zeiträume für welche die Bestimmungen des Vigilanzzustandes durchgeführt wird, derart verkürzt, dass zeitliche Änderungen des Systems nicht explizit bei der Modellierung der Übertragungsfunktion berücksichtigt werden müssen. Hierdurch ergibt sich in vorteilhafter Weise eine rechnerisch einfache Bestimmung des mindestens einen Parameters der parametrisierten Übertragungsfunktion, ohne dass ungewünschte Ungenauigkeiten bei der Modellierung der Übertragungsfunktion sich auf die Bestimmung des Vigilanzzustandes auswirken.

In einer weiteren Ausführungsform bildet die parametrisierte Übertragungsfunktion mindestens ein Totzeitglied ab. Hierbei kann ein Totzeitglied im Frequenzbereich, speziell im Laplace-Bereich, als

$$G_1(s) = e^{-sT_s} \hspace{5cm} \text{Formel 3}$$

dargestellt werden. Hierbei bezeichnet $T_s$ die Totzeit, wobei die Totzeit $T_s$ eine Zeitspanne zwischen einer Änderung am Systemeingang und einer Antwort am Systemausgang bezeichnet. Die Totzeit $T_s$ kann hierbei in vorteilhafter Weise eine Reaktionszeit des Kraftfahrzeugführers abbilden. Die Totzeit $T_s$ ist also als ein Parameter einer parametrisierten Übertragungsfunktion zu betrachten.

In einer weiteren Ausführungsform bildet die parametrisierte Übertragungsfunktion mindestens ein Tiefpassglied ab. Ein Tiefpassglied kann im Frequenzbereich, speziell im Laplace-Bereich, mittels

$$G_2 (s) = K/(1+Ts) \qquad\qquad \text{Formel 4}$$

beschrieben werden. Hierbei bezeichnet K einen Verstärkungsfaktor und T eine Zeitkonstante des Tiefpassgliedes. Hierdurch lässt sich in vorteilhafter Weise ein dynamisches Lenkverhalten des Kraftfahrzeugführers hinreichend gut zur Bestimmung des Vigilanzzustandes beschrieben. Insbesondere kann über eine Bestimmung des Verstärkungsfaktors K eine Heftigkeit der Reaktionen des Kraftfahrzeugführers bestimmt werden. Die Zeitkonstante T gibt Aufschluss über eine Geschwindigkeit des Lenkverhaltens.

Vorzugsweise bildet die parametrisierte Übertragungsfunktion ein Totzeitglied und ein Tiefpassglied ab. Selbstverständlich kann die parametrisierte Übertragungsfunktion auch weitere, komplexere Übertragungsglieder, beispielsweise Übertragungsglieder zweiter Ordnung, Proportionalglieder, Integrationsglieder und Differenzierungsglieder umfassen. Auch kann die parametrisierte Übertragungsfunktion eine Rückkopplung des Ausgangssignals, hier also des Reaktionssignals, zum Eingangssignal, hier also das Rauschsignal, abbilden. Hierdurch ergibt sich in vorteilhafter Weise, dass die real im Menschen vorhandene Kontrolle von motorischen Abläufen im Lenkverhalten noch besser beschrieben werden können. Insbesondere kann durch eine Abbildung einer Rückkopplung der Tatsache Rechnung getragen werden, dass in der Regel keine heftigen Lenkreaktionen von dem Kraftfahrzeugführer ausgeführt werden.

In einer weiteren Ausführungsform wird der mindestens eine Parameter kontinuierlich oder in regelmäßigen Zeitabständen oder zu ausgewählten Zeitpunkten bestimmt. Wird der mindestens eine Parameter kontinuierlich bestimmt, so ergibt sich in vorteilhafter Weise eine kontinuierliche Bestimmung des Vigilanzzustandes des Kraftfahrzeugführers. Für eine kontinuierliche Überwachung können hierbei beispielsweise Zeitintervalle mit einer vorbestimmten Zeitdauer des Rauschsignals und des Reaktionssignals zur Bestimmung des mindestens einen Parameters benutzt werden. Beispielsweise ist vorstellbar, dass für eine kontinuierliche Bestimmung des mindestens einen Parameters zeitliche Verläufe des Rauschsignals und des Reaktionssignals innerhalb eines vorbestimmten Zeitintervalls von beispielsweise 40 Sekunden vor dem aktuellen Zeitpunkt genutzt werden. Selbstverständlich ist es vorstellbar, dass das Zeitintervall eine kürzere oder längere Zeitdauer umfasst und/oder nicht direkt vor dem aktuellen Zeitpunkt, sondern eine vorbestimmte Zeitdauer vor dem aktuellen Zeitpunkt endet. Durch eine Bestimmung des mindestens einen Parameters in regelmäßigen Zeitabständen ergibt sich in vorteilhafter Weise eine Reduktion des Rechenaufwands zur Bestimmung des mindestens einen Parameters. Hierbei ist vorstellbar, dass der mindestens eine Parameter zum Beispiel jede Minute, alle 5 Minuten, alle 10 Minuten, alle 15 Minuten oder alle 30 Minuten bestimmt wird. Selbstverständlich sind auch andere Zeitabstände einer regelmäßigen Bestimmung oder gar keine kontinuierliche Bestimmung des mindestens einen Parameters vorstellbar. Durch eine Bestimmung des mindestens einen Parameters zu ausgewählten Zeitpunkten ergibt sich in vorteilhafter Weise eine weitere Reduktion des Rechenaufwands. Hierbei ist vorstellbar, dass eine Bestimmung des mindestens einen Parameters beispielsweise nach einem Bremsvorgang, nach einem Lenkvorgang, bei Über- oder Unterschreiten einer vorbestimmten Fahrzeuggeschwindigkeit oder weiteren fahrzeugdynamischen Vorgängen erfolgt.

In einer weiteren Ausführungsform ist das Rauschsignal ein niederfrequentes und/oder mittelwertfreies Rauschsignal. Ein menschlicher Kraftfahrzeugführer ist in der Regel nur in der Lage, niederfrequente Signale zu erfassen und zu kompensieren. Unter einem niederfrequenten Signal wird hierbei ein Signal mit einer oberen Grenzfrequenz von höchsten 20 Hz verstanden, vorzugsweise sollte eine obere Grenzfrequenz zwischen 2 und 10 Hz liegen. Hierdurch ergibt sich in vorteilhafter Weise, dass das Rauschsignal besonders geeignet ist, den mindestens einen Parameter und damit den Vigilanzzustand des Kraftfahrzeugführers zu bestimmen.

In einer weiteren Ausführungsform wird bei einem unkritischen Vigilanzzustand mindestens ein Verfahren zum Training kognitiver Informationsverarbeitungsprozesse des Kraftfahrzeugführers aktiviert. Alternativ oder kumulativ kann bei einem kritischen Vigilanzzustand ein Warnsignal generiert werden. Hierdurch wird in vorteilhafter Weise erreicht, dass Funktionen einer Informationsverarbeitung im Rahmen einer Fahrzeugführung insbesondere bei älteren Kraftfahrzeugführern durch Training verbessert werden können. Hierbei können insbesondere Defizite im Bereich einer parallelen Verarbeitung von Informationen, das heißt bei der so genannten geteilten Aufmerksamkeit, kompensiert werden. Das Training hält den Fahrer aktiviert, so dass er in monotonen Situationen nicht in einen kritischen Vigilanzzustand gerät.

Beispielsweise kann eine Applikation kognitiver Trainingsprogramme während monotoner Streckenabschnitte mit geringen kognitiven Anforderungen und/oder auf Fahrten mit wenig Lenkanforderungen erfolgen. Hierbei sind Fahrten und/oder Streckenabschnitte mit geringen kognitiven Anforderungen beispielsweise folgendermaßen definiert:

- Streckenabschnitte, die nicht länger als 300 km sind,
- niedriges Verkehrsaufkommen,
- möglichst optimale Beleuchtungs- und Witterungsverhältnisse, insbesondere Fahrten bei Tageslicht, Fahrten ohne Nebel, ohne Regen, ohne Schnee und ohne Sonnenblendung,
- einfach Streckenanforderungen, zum Beispiel möglichst wenig Kurven und/oder wenig Baustellen.

Hierbei liegt es im Ermessen des Fachmanns, geeignete Systeme zur Detektion geeigneter Streckenabschnitte für eine Applikation kognitiver Trainingsprogramme zu schaffen.

Eine weitere Voraussetzung für die Applikation kognitiver Trainingsprogramme ist, dass das beschriebene Verfahren zur Bestimmung des Vigilanzzustandes eine durchschnittlich bis sehr gute Vigilanz des Kraftfahrzeugführers bestimmt.

Weiter kann die Applikation kognitiver Trainingsprogramme vom Kraftfahrzeugführer an- und abschaltbar sein. Weiter kann die Applikation kognitiver Trainingsprogramme unterbrechbar sein, insbesondere wenn überraschende oder schwierige Fahrsituationen auftreten, die eine ungeteilte Aufmerksamkeit des Kraftfahrzeugführers beanspruchen.

Kognitive Trainingsprogramme können beispielsweise folgendermaßen ausgestaltet sein:

- Reaktionsaufgaben auf Signale, beispielsweise akustische und/oder visuelle Signale. Bei akustischen Signalen ergibt sich in vorteilhafter Weise, dass eine gleichzeitige Verteilung kognitiver Ressourcen des Kraftfahrzeugführers auf die Fahraufgabe und das Trainingsprogramm möglich ist. Bei einer Reaktion auf visuelle Signale ist es erforderlich, dass die visuellen Signale sich auf eine aktuelle Verkehrssituation beziehen, da keine Verteilung kognitiver Ressourcen möglich ist. Zum Beispiel kann eine Reaktionsaufgabe darin bestehen, auf eine bestimmte Fahrzeugklasse oder bestimmte Fahrmanöver des eigenen oder fremder Fahrzeuge zu reagieren.
- Denkaufgaben über sprachliche Bedienoberflächen,
- Aufgaben im Zusammenhang mit der Fahraufgabe, zum Beispiel beim Überholen eines Fahrzeugs unter Beachtung sämtlicher Anforderungen (Schulterblick, Beschleunigung, usw.).

In besonders bevorzugter Weise sollten die kognitiven Trainingsprogramme einen spielerischen Ehrgeiz des Kraftfahrzeugführers wecken. Hierdurch ergibt sich in vorteilhafter Weise, dass der Kraftfahrzeugführer die Aufgaben eines kognitiven Trainingsprogramms wiederholt durchführt.

Weiter vorgeschlagen wird eine Vorrichtung zur Lenkung eines Kraftfahrzeugs, wobei die Lenkung mindestens eine Einheit zur Erzeugung eines Rauschsignals und mindestens eine Einheit zur Aufbringung von mindestens einem von dem Rauschsignal abhängigen Zusatzmoment auf die Lenkung, insbesondere eine Lenksäule des Kraftfahrzeugs umfasst. Weiter umfasst die Lenkung mindestens eine Einheit zur Bestimmung eines Reaktionssignals eines Kraftfahrzeugführers und mindestens eine Einheit zur Systemidentifikation. Hierbei ist mittels der vorgeschlagenen Vorrichtung mindestens eines der vorhergehend beschriebenen Verfahren durchführbar.

Die Erfindung wird anhand eines Ausführungsbeispiels näher erläutert. Die Figuren zeigen:

Figur 1    einen schematischen Signalverlauf eines Verfahrens zur Bestimmung eines Vigilanzzustandes und

Figur 2    ein schematisches Blockschaltbild einer Lenkung eines Kraftfahrzeuges.

Im Folgenden bezeichnen gleiche Bezugszeichen Elemente mit gleichen oder ähnlichen technischen Eigenschaften.

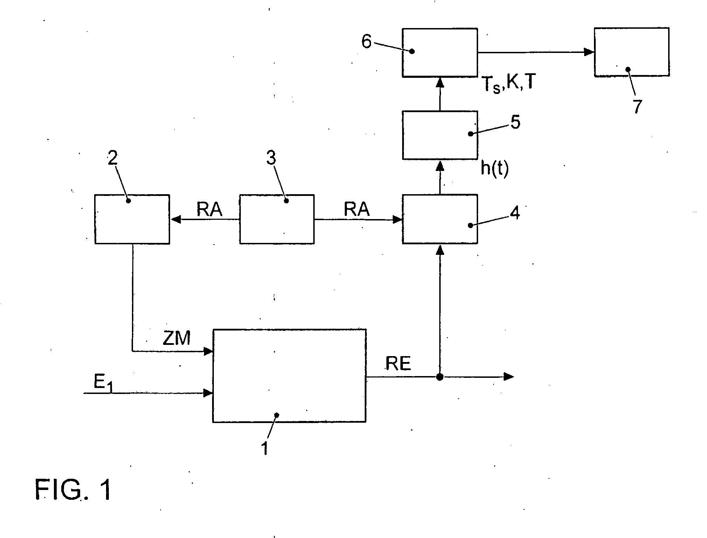

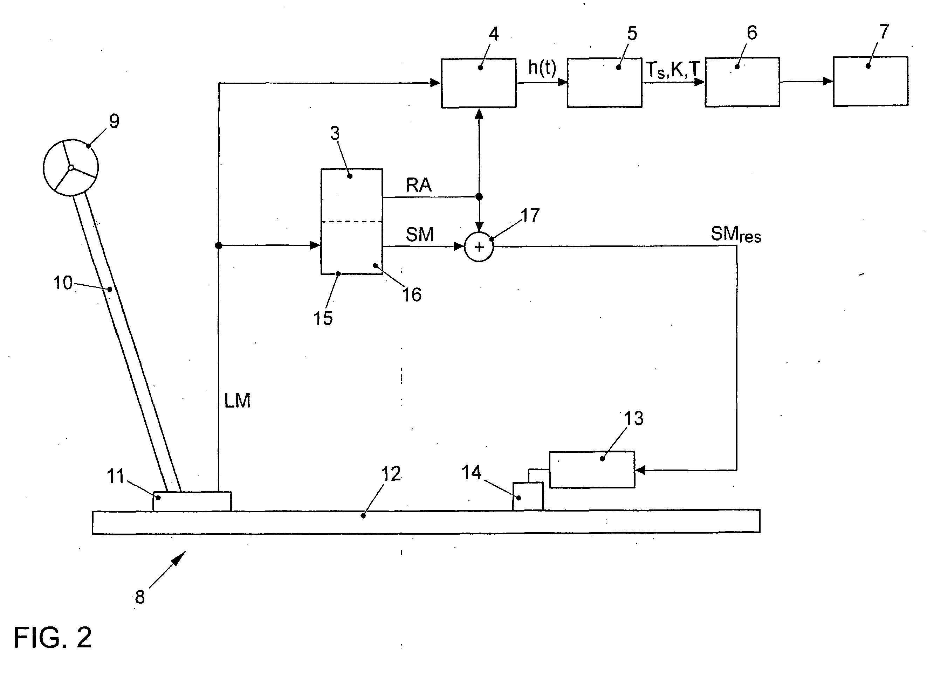

Figur 1 zeigt einen schematischen Signalverlauf in einem Verfahren zur Bestimmung eines Vigilanzzustands eines Kraftfahrzeugführers 1. Hierbei erfasst der Kraftfahrzeugführer 1 zwei Eingangssignale. Ein erstes Eingangssignal E1 setzt sich aus einer Summe von Lenkbewegungen zusammen, die aus einer Fahraufgabe resultieren. Lenkbewegungen resultieren hierbei aus einem Navigationswunsch des Kraftfahrzeugführers 1, durchgeführten Fahrmanövern, wie zum Beispiel einem Überholmanöver, und Lenkbewegungen zur Fahrzeugstabilisierung. Ein zweites Eingangssignal für den Kraftfahrzeugführer 1 ist ein auf die in Figur 2 dargestellte Lenksäule 10 aufgebrachtes Zusatzmoment ZM. Das Zusatzmoment ZM wird hierbei von einer Einheit 2 zur Erzeugung des Zusatzmoments ZM erzeugt. Die Einheit 2 zur Erzeugung des Zusatzmoments ZM kann beispielsweise der in Figur 2 dargestellte Servomotor 13 sein. Die Einheit 2 zur Erzeugung des Zusatzmoments ZM erzeugt das Zusatzmoment ZM in Abhängigkeit eines Rauschsignals RA. Das Rauschsignal RA wird von einer Einheit 3 zur Erzeugung eines Rauschsignals RA erzeugt. Der Kraftfahrzeugführer 1 erzeugt in Abhängigkeit des ersten Eingangssignals E1 und des Zusatzmoments ZM ein Reaktionssignal RE. Das Rauschsignal RA und das Reaktionssignal RE werden einer Einheit 4 zur Korrelation datentechnisch übermittelt. Die Einheit 4 zur Korrelation bestimmt eine Impulsantwort h(t) beispielsweise mittels einer Kreuzkorrelation des Rauschsignals RA mit dem Reaktionssignals RE und einer Autokorrelation des Rauschsignals RA. Eine Einheit 5 zur Systemidentifikation bestimmt aus der Impulsantwort h(t) und einer vorbestimmten parametrisierten Übertragungsfunktion die Parameter der Übertragungsfunktion. Die Übertragungsfunktion kann hierbei beispielsweise ein Totzeitglied und ein Tiefpassglied abbilden. Als Parameter umfasst die Übertragungsfunktion hierbei eine Totzeit $T_s$, einen Verstärkungsfaktor K und eine Zeitkonstante T. Eine Einheit 6 zur Bestimmung des Vigilanzzustandes bestimmt in Abhängigkeit einer absoluten und/oder relativen Änderung der Totzeit $T_s$, des Verstärkungsfaktors K und der Zeitkonstante T den Vigilanzzustand des Kraftfahrzeugführers 1. Detektiert die Einheit 6 zur Bestimmung des Vigilanzzustandes einen kritischen Vigilanzzustand des Kraftfahrzeugführers 1, so kann die Einheit 6 zur Bestimmung des Vigilanzzustandes eine Einheit 7 zur Warnung des Kraftfahrzeugführers 1 aktivieren. Die Einheit 7 zur Warnung des Kraftfahrzeugführers 1 erzeugt hierbei beispielsweise ein akustisches Warnsignal.

In Figur 2 ist ein schematisches Blockschaltbild einer Vorrichtung 8 zur Lenkung eines nicht dargestellten Kraftfahrzeugs dargestellt. Die Vorrichtung 8 umfasst ein Lenkrad 9, eine mechanisch mit dem Lenkrad 9 gekoppelte Lenksäule 10, einen Sensor 11 zur Erfassung eines Lenkmoments LM, eine Lenkzahnstange 12 und einen Servomotor 13, der über ein Getriebe 14 mit der Lenkzahnstange 12 gekoppelt ist. Weiter umfasst die Vorrichtung 8 ein Lenkungssteuergerät 15. Das Lenkungssteuergerät 15 umfasst die in Figur 1 dargestellte Einheit 3 zur Erzeugung eines Rauschsignals RA. Weiter umfasst das Lenkungssteuergerät 15 eine Einheit 16 zur Berechnung eines Servomoments SM. Die Einheit 16 zur Berechnung eines Servomoments SM berechnet ein Servomoment SM mittels einer Berechnungsvorschrift aus dem Lenkmoment LM. Das erfasste Lenkmoment LM umfasst auch ein vom Kraftfahrzeugführer 1 mittels des Lenkrads 9 auf die Lenksäule 10 aufgebrachtes Handmoment. Das von der Einheit 3 zur Erzeugung eines Rauschsignals RA erzeugte Rauschsignal RA wird mittels einer Additionseinheit 17 auf das Servomoment SM addiert. Das hieraus resultierende Servomoment $SM_{res}$ dient als Sollwert für den Servomotor 13. Der Servomotor 13 erzeugt in Abhängigkeit des resultierenden Servomoments $SM_{res}$ ein Moment, welches er mittels des Getriebes 14 auf die Lenkzahnstange 12 aufbringt. Hierbei dient also der Servomotor 13 als Einheit 2 zur Aufbringung eines Zusatzmoments ZM (siehe Figur 1), wobei das Zusatzmoment ZM vom zeitlichen Verlauf des Rauschsignals RA abhängt. Das von dem Rauschsignal RA abhängige Zusatzmoment ZM wird über die Lenkzahnstange 12 auf die Lenksäule 10 übertragen. Der Sensor 11 zur Erfassung des Lenkmoments LM erfasst dann ein Handmoment resultierend aus Lenkbewegungen des Kraftfahrzeugführers 1, die einerseits auf den vorhergehend beschriebenen Lenkbewegungen bestehen und andererseits zur Kompensation des Zusatzmoments ZM dienen. Das Lenkmoment LM und das von der Einheit 3 zur Erzeugung eines Rauschsignals RA erzeugte Rauschsignal RA werden datentechnisch an eine Einheit 4 zur Korrelation übermittelt. In analoger Weise zu dem in Figur 1 beschriebenem Verfahren zur Bestimmung eines Vigilanzzustandes eines Kraftfahrzeugführers 1 berechnet die Einheit 4 zur Korrelation eine Impulsantwort h(t). Eine Einheit 5 zur Systemidentifikation identifiziert eine Totzeit $T_s$, einen Verstärkungsfaktor K und eine Zeitkonstante T. Eine Einheit 6 zur Bestimmung des Vigilanzzustandes bestimmt in Abhängigkeit der erfassten Parameter $T_s$, K, T einen kritischen oder unkritischen Vigilanzzustand und kann bei Bedarf eine Einheit 7 zur Warnung des Kraftfahrzeugführers 1 aktivieren. In der in Figur 2 gezeigten Ausführungsform entspricht der Sensor 11 zur Erfassung des Lenkmoments der mindestens einen Einheit zur Bestimmung eines Reaktionssignals RA des Kraftfahrzeugführers 1.

**Bezugszeichenliste**

| | |
|---|---|
| 1 | Kraftfahrzeugführer |
| 2 | Einheit zur Erzeugung eines Zusatzmoments |
| 3 | Einheit zur Erzeugung eines Rauschsignals |
| 4 | Einheit zur Korrelation |
| 5 | Einheit zur Systemidentifikation |
| 6 | Einheit zur Bestimmung eines Vigilanzzustandes |
| 7 | Einheit zur Warnung eines Kraftfahrzeugführers |
| 8 | Vorrichtung zur Lenkung eines Kraftfahrzeugs |
| 9 | Lenkrad |
| 10 | Lenksäule |
| 11 | Sensor zur Erfassung eines Lenkmoments |
| 12 | Lenkzahnstange |
| 13 | Servomotor |
| 14 | Getriebe |
| 15 | Lenkungssteuergerät |
| 16 | Einheit zur Berechnung eines Servomoments |
| E1 | erstes Eingangssignal |

| | |
|---|---|
| ZM | Zusatzmoment |
| RA | Rauschsignal |
| RE | Reaktionssignal |
| h(t) | Impulsantwort |
| $T_s$ | Totzeit |
| K | Verstärkungsfaktor |
| T | Zeitkonstante |
| SM | Servomoment |
| $SM_{res}$ | resultierendes Servomoment |
| LM | Lenkmoment |

**Patentansprüche**

1. Verfahren zur Bestimmung eines Vigilanzzustandes eines Kraftfahrzeugführers (1), wobei

- ein Rauschsignal (RA) erzeugt
- ein Reaktionssignal (RE) des Kraftfahrzeugführers (1) bestimmt wird
- und der Vigilanzzustand aus einem zeitlichen Verlauf des Rauschsignals (RA) und einem zeitlichen Verlauf des Reaktionssignals (RE) bestimmt wird,

**dadurch gekennzeichnet, dass**
ein von dem Rauschsignal (RA) abhängiges Zusatzmoment (ZM) auf eine Lenkung aufgebracht wird und mindestens ein Parameter einer parametrisierten Übertragungsfunktion von Rauschsignal (RA) zu Reaktionssignal (RE) bestimmt wird, wobei der Vigilanzzustand abhängig von einem absoluten Wert des mindestens einen Parameters und/oder abhängig von einer relativen Änderung des mindestens einen Parameters zu mindestens einem früheren Parameter bestimmt wird, wobei der mindestens eine frühere Parameter zu einem früheren Zeitpunkt bestimmt wurde.

2. Verfahren nach Anspruch 1, **dadurch gekennzeichnet, dass** der mindestens eine Parameter der parametrisierten Übertragungsfunktion mittels einer Kreuzkorrelation ($R_{XY}$) des Rauschsignals (RA) mit dem Reaktionssignal (RE) bestimmt wird.

3. Verfahren nach Anspruch 1, **dadurch gekennzeichnet, dass** der mindestens eine Parameter der parametrisierten Übertragungsfunktion mittels einer Autokorrelation ($R_{XX}$) des Rauschsignals (RA) und/oder einer Autokorrelation ($R_{YY}$) des Reaktionssignals (RE) bestimmt wird.

4. Verfahren nach einem der vorangegangenen Ansprüche, **dadurch gekennzeichnet, dass** die parametrisierte Übertragungsfunktion eine lineare und/oder zeitinvariante Übertragungsfunktion ist.

5. Verfahren nach einem der vorangegangenen Ansprüche, **dadurch gekennzeichnet, dass** die parametrisierte Übertragungsfunktion mindestens ein Totzeitglied ($G_1$) abbildet.

6. Verfahren nach einem der vorangegangenen Ansprüche, **dadurch gekennzeichnet, dass** die parametrisierte Übertragungsfunktion mindestens ein Tiefpassglied ($G_2$) abbildet.

7. Verfahren nach einem der vorangegangenen Ansprüche, **dadurch gekennzeichnet, dass** der mindestens eine Parameter kontinuierlich oder in regelmäßigen Zeitabständen oder zu ausgewählten Zeitpunkten bestimmt wird.

8. Verfahren nach einem der vorangegangenen Ansprüche, **dadurch gekennzeichnet, dass** das Rauschsignal ein niederfrequentes und/oder mittelwertfreies Rauschsignal ist.

9. Verfahren nach einem der vorangegangenen Ansprüche, **dadurch gekennzeichnet, dass** bei einem unkritischen Vigilanzzustand mindestens ein Verfahren zum Training kognitiver Informationsverarbeitungsprozesse des Kraftfahrzeugführers aktiviert wird und/oder bei einem kritischen Vigilanzzustand ein Warnsignal generiert wird.

10. Vorrichtung (8) zur Lenkung eines Kraftfahrzeugs, wobei die Lenkung mindestens eine Einheit (3) zur Erzeugung eines Rauschsignals (RA), mindestens eine Einheit (11) zur Bestimmung eines Reaktionssignals (RE) eines Kraftfahrzeugführers (1) und mindestens eine Einheit (5) zur Systemidentifikation umfasst,

**dadurch gekennzeichnet, dass**

die Vorrichtung weiterhin mindestens eine Einheit (2, 13) zur Aufbringung von mindestens einem von dem Rauschsignal (RA) abhängigen Zusatzmoment (ZM) auf die Lenkung umfasst und

mittels der mindestens einen Einheit (5) zur Systemidentifikation mindestens ein Parameter einer parametrisierten Übertragungsfunktion von Rauschsignal (RA) zu Reaktionssignal (RE) bestimmbar ist.

**Claims**

1. Method for determining a state of vigilance of a motor vehicle driver (1), wherein

   - a noise signal (RA) is generated,
   - a reaction signal (RE) of the motor vehicle driver (1) is determined,
   - and the state of vigilance is determined from a time profile of the noise signal (RA) and a time profile of the reaction signal (RE),

   **characterized in that**
   an additional torque (ZM) which is dependent on the noise signal (RA) is applied to a steering system, and at least one parameter of a parameterised transmission function of noise signal (RA) to reaction signal (RE) is determined, wherein the state of vigilance is dependent on an absolute value of the at least one parameter and/or dependent on a relative change in the at least one parameter with respect to at least one earlier parameter, wherein the at least one earlier parameter was determined at an earlier time.

2. Method according to Claim 1, **characterized in that** the at least one parameter of the parameterised transmission function is determined by means of a cross-correlation ($R_{XY}$) of the noise signal (RA) with the reaction signal (RE).

3. Method according to Claim 1, **characterized in that** the at least one parameter of the parameterised transmission function is determined by means of an auto-correlation ($R_{XX}$) of the noise signal (RA) and/or an auto-correlation ($R_{YY}$) of the reaction signal (RE).

4. Method according to one of the preceding claims, **characterized in that** the parameterised transmission function is a linear and/or time-invariant transmission function.

5. Method according to one of the preceding claims, **characterized in that** the parameterised transmission function maps at least one delay element ($G_1$).

6. Method according to one of the preceding claims, **characterized in that** the parameterised transmission function maps at least one low-pass element ($G_2$).

7. Method according to one of the preceding claims, **characterized in that** the at least one parameter is determined continuously or at regular time intervals or at selected times.

8. Method according to one of the preceding claims, **characterized in that** the noise signal is a low-frequency and/or mean-value-free noise signal.

9. Method according to one of the preceding claims, **characterized in that** in an uncritical state of vigilance at least one method for training cognitive information-processing processes of the motor vehicle driver is activated and/or in a critical state of vigilance a warning signal is generated.

10. Device (8) for steering a motor vehicle, wherein the steering system comprises at least one unit (3) for generating a noise signal (RA), at least one unit (11) for determining a reaction signal (RE) of a motor vehicle driver (1) and at least one unit (5) for system identification,
    **characterized in that**
    the device also comprises at least one unit (2, 13) for applying at least one additional torque (ZM) dependent on the noise signal (RA), to the steering system, and at least one parameter of a parameterised transmission function of noise signal (RA) to reaction signal (RE) can be determined by means of the at least one unit (5) for system identification.

**Revendications**

1. Procédé de détermination d'un état de vigilance d'un conducteur de véhicule automobile (1) :

   - un signal sonore (RA) étant produit ;
   - un signal de réaction (RE) du conducteur de véhicule automobile (1) étant déterminé ; et

   l'état de vigilance étant déterminé à partir d'une courbe dans le temps du signal sonore (RA) et d'une courbe dans le temps du signal de réaction (RE) ; **caractérisé en ce que** :

   un couple supplémentaire (ZM) dépendant du signal sonore (RA) est appliqué à une direction et au moins un paramètre d'une fonction de transmission paramétrée est déterminé par le signal sonore (RA) pour être transformé en signal de réaction (RE), l'état de vigilance étant déterminé en fonction d'une valeur absolue de l'au moins un paramètre et/ou en fonction d'une variation relative de l'au moins un paramètre par rapport à au moins un paramètre précédent, l'au moins un paramètre précédent ayant été déterminé à un moment précédent.

2. Procédé selon la revendication 1, **caractérisé en ce que** l'au moins un paramètre de la fonction de transmission paramétrée est déterminé au moyen d'une corrélation croisée ($R_{xy}$) du signal sonore (RA) avec le signal de réaction (RE).

3. Procédé selon la revendication 1, **caractérisé en ce que** l'au moins un paramètre de la fonction de transmission paramétrée est déterminé au moyen d'une corrélation automatique ($R_{xx}$) du signal sonore (RA) et/ou d'une corrélation automatique ($R_{yy}$) du signal de réaction (RE).

4. Procédé selon l'une quelconque des revendications précédentes, **caractérisé en ce que** la fonction de transmission paramétrée est une fonction de transmission linéaire et/ou invariable dans le temps.

5. Procédé selon l'une quelconque des revendications précédentes, **caractérisé en ce que** la fonction de transmission paramétrée reproduit au moins un élément à temps mort ($G_1$).

6. Procédé selon l'une quelconque des revendications précédentes, **caractérisé en ce que** la fonction de transmission paramétrée reproduit au moins un élément passe-bas ($G_2$).

7. Procédé selon l'une quelconque des revendications précédentes, **caractérisé en ce que** l'au moins un paramètre est déterminé en continu ou à intervalles réguliers ou à des moments choisis.

8. Procédé selon l'une quelconque des revendications précédentes, **caractérisé en ce que** le signal sonore est un signal sonore à basse fréquence et/ou exempt de valeur centrale.

9. Procédé selon l'une quelconque des revendications précédentes, **caractérisé en ce qu'**en présence d'un état de vigilance non critique, au moins un procédé d'entraînement des processus de traitement de l'information cognitifs du conducteur de véhicule automobile est activé et/ou qu'un signal d'avertissement est généré en cas d'état de vigilance critique.

10. Dispositif (8) de direction d'un véhicule automobile, la direction comprenant au moins une unité (3) servant de production d'un signal sonore (RA), au moins une unité (11) de détermination d'un signal de réaction (RE) d'un conducteur de véhicule automobile (1) et au moins une unité (5) d'identification systémique ; **caractérisé en ce que** :

    le dispositif comprend en outre au moins une unité (2, 13) permettant d'appliquer au moins un couple supplémentaire (ZM) à la direction en fonction du signal sonore (RA) ; et
    au moins un paramètre d'une fonction de transmission paramétrée pouvant être déterminée par le signal sonore (RA) pour former le signal de réaction (RE) à l'aide de l'au moins une unité (5) d'identification systémique.

FIG. 1

FIG. 2

**IN DER BESCHREIBUNG AUFGEFÜHRTE DOKUMENTE**

**In der Beschreibung aufgeführte Patentdokumente**

- EP 1257202 B1
- EP 1929950 A1